(19) Europäisches Patentamt
European Patent Office
Office européen des brevets

(11) **EP 0 525 119 B1**

(12) **EUROPEAN PATENT SPECIFICATION**

(45) Date of publication and mention
of the grant of the patent:
**18.03.1998 Bulletin 1998/12**

(21) Application number: **91909732.9**

(22) Date of filing: **19.04.1991**

(51) Int. Cl.[6]: **A61K 38/21**, A61K 38/00,
A61K 39/44, A61K 47/48

(86) International application number:
**PCT/US91/02696**

(87) International publication number:
**WO 91/16071 (31.10.1991 Gazette 1991/25)**

(54) **ANTIBODY CONJUGATES FOR TREATMENT OF NEOPLASTIC DISEASE**

ANTIKÖRPERKONJUGATE FÜR DIE BEHANDLUNG NEOPLASTISCHER KRANKHEITEN

CONJUGUES D'ANTICORPS PERMETTANT DE SOIGNER LES MALADIES NEOPLASIQUES

(84) Designated Contracting States:
**AT BE CH DE DK ES FR GB GR IT LI LU NL SE**

(30) Priority: **19.04.1990 US 510923**

(43) Date of publication of application:
**03.02.1993 Bulletin 1993/05**

(73) Proprietor:
**RESEARCH DEVELOPMENT FOUNDATION
(a Nevada corporation)
Carson City Nevada 89703 (US)**

(72) Inventor: **ROSENBLUM, Michael, G.
Houston, TX 77071 (US)**

(74) Representative:
**Wilkinson, Stephen John et al
Stevens, Hewlett & Perkins
1 St. Augustine's Place
Bristol BS1 4UD (GB)**

(56) References cited:
EP-A- 350 230          EP-A- 396 387
US-A- 4 590 071          US-A- 4 863 726
US-A- 4 888 415          US-A- 4 894 225
US-A- 4 894 227          US-A- 4 894 443
US-A- 5 017 371

- MOL. BIOTHER., vol. 3, no. 1, March 1991, pages 6-13; M.G. ROSENBLUM ET AL.: 'A SPECIFIC AND POTENT IMMUNOTOXIN COMPOSED OF ANTIBODY ZME-018 AND THE PLANT TOXIN GELONIN'

- PROC. AM. ASSOC. CANCER RES. ANNU. MEET., vol. 29, 1988, page 427; M.G. ROSENBLUM ET AL.: 'AN ANTIMELANOMA IMMUNOTOXIN COMPOSED OF ANTIBODY ZME-018 AND THE PLANT TOXIN GELONIN'
- PROC. AM. ASSOC. CANCER RES. ANNU. MEET., vol. 28, 1987, page 384; J.E. ZUCKERMAN ET AL.: 'PREPARATION AND BIOLOGICAL ACTIVITY OF RECOMBINANT LEUKOCYTE INTERFERON CONJUGATED TO AN ANTIMELANOMA MURINE MONOCLONAL ANTIBODY (ZME-018)
- CANCER RESEARCH, vol. 47, 1987, pages 3169-3173; G. SIVAM ET AL.: 'IMMUNOTOXINS TO A HUMAN MELANOMA-ASSOCIATED ANTIGEN: COMPARISON OF GELONIN WITH RICIN AND OTHER A CHAIN CONJUGATES'
- NCI MONOGRAPHS, vol. 3, 1987, pages 3-9; J.L. MURRAY ET AL.: 'RADIOIMMUNOIMAGING IN MALIGANT MELANOMA PATIENTS WITH THE USE OF INDIUM-111- LABELED ANTIMELANOMA MONOCLONAL ANTIBODY (ZME-018) TO HIGH-MOLECULAR-WEIGHT ANTIGEN'
- J. Nucl. Med., Volume 28, issued September 1987; S. MING CHAN et al.: "Comparison of gallium-67 versus indium-III monoclonal antibody (96.5. ZME-018) in detection of human melanoma in athymic mice", pages 1441-1446

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 525 119 B1

- Biological Abstracts, Volume 84, No. 10, issued 1987; KIRKWOOD et al.: "Scintigraphic detection of metastatic melanoma using indium-III DTPA conjugated anti-GP240 antibody (ZME-018)", see page 628, column 1, Abstract No. 99695, J. Clin. Oncol. 5 (8):1247-1255
- Cancer Research, Volume 50, No. 19, issued 01 October 1990; PEARSON et al.: "Enhanced therapeutic efficacy against an ovarian tumor xenograft of immunotoxins used in conjunction with recombinant alpha-interferon", pages 6379-6388
- Cancer Research, Volume 50, No. 1, issued 01 January 1990; YOKOTA et al.: "Synergistic potentiation of invivo antitumor activity of anti-human T-leukemia immunotoxins by recombinant alpha-interferon and daunorubicin", pages 32-37

**Description**

Field of the Invention

The present invention relates generally to the field of immunoconjugates and, more particularly, to the use of immunoconjugates in the treatment of cancer. The invention also relates to the treatment of melanoma with conjugates of monoclonal antibodies (MoAbs) and cytotoxic moieties such as gelonin, a ribosomal inhibiting protein, other plant-derived cytotoxic moieties, or cytotoxic or cytostatic biological response modifiers.

Background of the Invention

The necessity for precisely targeting cancer therapy is critical since adequate tumor response is dependent upon delivery and maintenance of intratumor therapeutic concentrations of drugs. Site-directed therapy has become a goal of several investigators utilizing monoclonal antibodies as specific carriers of therapeutic agents.

Cancer is one of the leading causes of mortality and morbidity in the western world. There are many types of cancer, each with its own characteristics. However, cancers share at least one characteristic in common, they involve defects in the cellular growth regulatory process.

Melanoma, the most virulent of skin cancers, is a highly metastatic disease affecting both sexes and is almost uniformly fatal within five years of diagnosis. Surgical removal of localized malignancies has proven effective only when the disease has not spread beyond the primary lesion. Once the disease has spread, the surgical procedures must be supplemented with other more general procedures to eradicate the diseased or malignant cells. Most of the commonly utilized supplementary procedures such as irradiation or chemotherapy are not localized to the tumor cells and, although they have a proportionally greater destructive effect on malignant cells, often affect normal cells to some extent.

Many tumors express antigens or antigenic determinants which are either expressed very weakly or not expressed at all by normal cells. Some tumor cells express antigens which are expressed by embryonic cell types but are not expressed by normal cells of a mature animal. These abnormally expressed antigens are known as tumor-associated antigens. These antigens are specific in that while a particular antigen may be expressed by more than one tumor, it is usually expressed by all or most cells of the particular tumors which express it. A tumor cell may express one or more than one tumor-associated antigen. These tumor-associated antigens may be expressed on the surface of the cell (cell surface antigen), may be secreted by the tumor cell (secreted antigens) or may remain inside the cell (intracellular antigen).

The presence of these tumor-associated antigens has been utilized to detect, diagnose and localize the tumor. In some cases the presence of the tumor-associated antigens on the tumor cells has allowed the targeting of specific drugs and other treatment means specifically to the tumor cells.

Antibodies are proteins normally produced by the immune system of an animal in response to foreign antigens or antigenic determinants. Antibodies bind to the specific antigen to which they are directed. For instance, antibodies to other melanoma antigens have been utilized to demonstrate specific tumor localization in man after systemic and intraperitoneal administration.

One method of targeting chemotherapeutic agents to tumor cells and to diminish their effects on normal cells has been made possible with the development of MoAbs directed against antigens on the tumor cells which do not occur on normal cells. Monoclonal antibodies directed to specific antigens or antigenic determinants may be prepared in large quantities.

Antibodies may be labelled in order to allow their use for localization and treatment of malignant diseases. Such radiolabelled monoclonal antibodies to tumor cell surface antigens have been successfully utilized to image tumors in patients by external scintography (Deland, Semin. Nucl. Med. 19(3): 158-65 (Review) (1989); Juhl, Hepatogastroenterology 36(1): 27-32 (Review), (1989)). Antibodies, coupled to drugs, may be used as a delivery system by which the drug is targeted to a specific tumor cell type against which the antibody is directed because of the antibodies' unique ability to localize at the tumor site after systemic administration. Antibodies may also be coupled to toxins and thus act as a delivery system to target the toxins directly to specific tumor cells.

The cytotoxic agents frequently utilized for antibody conjugates primarily fall into three classes of agents: toxins, radionuclides and chemotherapeutic agents. Antibody conjugates with each of these types of agents offer substantial promise as therapeutic agents but present some unique problems as well (Frankel, et al. Ann. Rev. Med. 37: 125-142 (1986); Reimann et al., J. Clin. Invest. 82(1): 129-138 (1988).). Immunoconjugates containing plant toxins also offer a unique advantage to other types of antibody conjugates because:

1. Doses of immunotoxins required for antitumor activity are, in general, much lower than that required for antibody-drug conjugates.

2. The conjugation of toxins to antibodies does not appear to affect antibody affinity.

Gelonin is a glycoprotein (M.W. approximately 29-30,000 Kd) purified from the seeds of Gelonium multiforum. Gelonin belongs to a class of potent ribosomal-inactivating plant toxins. Other members of this class of ribosomal-inactivating plant toxins are the chains of abrin, ricin and modeccin. Gelonin, like abrin and ricin, inhibits protein synthesis by damaging the 60S sub-unit of mammalian ribosomes. Although the A chain of ricin (RTA) has been popular for use in immunotoxins, gelonin appears to be more stable to chemical and physical treatment than RTA (Barbieri et al., Cancer Surv. 1: 489-520 (1982)). Furthermore, gelonin itself does not bind to cells and is, therefore, non-toxic (except in high concentrations) and is safe to manipulate in the laboratory. The inactivation of ribosomes is irreversible, does not appear to involve co-factors and occurs with an efficiency which suggests that gelonin acts enzymatically.

Numerous prior workers have suggested or reported linking cytotoxic agents to antibodies to make "immunotoxins." Of particular interest have been immunotoxins of monoclonal antibodies conjugated to the enzymatically active portions (A chains) of toxins of bacterial or plant origin such as ricin or abrin (Nevelle et al., Immunol. Rev., 62: 75-92 (1982); Ross et al., European J. Biochem. 104 (1980); Vitteta et al., Immunol. Rev. 62: 158-183 (1982); Ross et al., Cancer Res. 42: (1982) 457-464; Trowbridge and Domingo, Nature (Cond.) 294: 171-173 (1981)).

Gelonin and ricin are among the most active toxins which inhibit protein synthesis on a protein weight basis. Gelonin is 10 to 1000 times more active in inhibiting protein synthesis than ricin A chain. Peptides like ricin and abrin are composed of two chains, an A chain which is the toxic unit and a B chain which acts by binding to cells. Unlike ricin and abrin, gelonin is composed of a single chain, and, because it lacks a B chain for binding to cells, it is itself relatively non-toxic to intact cells (Stirpe, et al. J. Biol. Chem. 255: 6947-6953 (1980)). Mammalian cells apparently lack the ability to bind and/or to internalize the native gelonin molecule. Conjugates of gelonin with a tumor-targeting monoclonal antibody, such as the monoclonal antibody ZME directed to an antigen present on certain tumor cells such as melanoma cells, provide both a specific method for binding the gelonin to the cell and a route for internalization of the gelonin-antibody complex. One of the advantages of using the toxin gelonin over using toxins such as ricin A chain is this reduced toxicity to normal tissues as compared to the ricin A chain. Gelonin-coupled with a monoclonal antibody directed to an anti-tumor associated antigen is an active and selective immunotoxic agent for tumor therapy.

Previous studies have described a number of antibody-toxin conjugates containing gelonin (Lambert et al., J. Biol. Chem. 260: 12035-12038 (1985); Thorpe et al., Eur. J. Biochem 116: 447-454 (1981); Singh et al., J. Biol. Chem. 264: 3089-95 (1989); Scott et al., J. Natl. Cancer Inst. 79: 1163-72 (1987); Tedder et al., J. Immunol. 137(4): 1387-91 (1986)). Recently Ozawa, et al. (Int. J. Cancer 43: 152-157) have constructed a gelonin immunotoxin comprised of antibody B467 which binds to the cellular receptor for epidermal growth factor (EGF). This B467-gelonin conjugate was highly cytotoxic for EGF receptor expressing cells but was non-cytotoxic for receptor-deficient cells. Sivam, et al. (Cancer Research 47: 3169-3173 (1987)) have made a conjugate of the antimelanoma antibody 9.2.2.7 with gelonin and compared in vitro and in vivo cytotoxic activity with a 9.2.2.7 conjugate of abrin and ricin A chain. These studies demonstrated that gelonin conjugates show substantial cytotoxic effects selectively against antigen-positive cells in vitro. In vivo experiments demonstrated that gelonin conjugates are not toxic up to 2 mg total antibody dose/mouse and that multiple I.V. administration of gelonin immunotoxin significantly retarded the growth of an established subcutaneous human tumor xenograft in nude mice. Compared to conjugates with abrin and ricin, gelonin conjugates appeared to have similar potency, better selectively and tumor localization with more significant in vivo therapeutic properties.

Since the antibody to which the drug, toxin or radioactive label is coupled binds only to tumor cells expressing a specific antigen, only the tumor cells are killed. Conversely, with radiation therapy, radiation from the radiolabelled compounds is not limited solely to the tumor cells in which the radiation is taken up. For example, metabolic or enzymatic degradation of the antibody may release the radiolabel and allow it to distribute to other tissues such as kidneys or bone marrow causing unacceptable radiation damage to these organs. Radiolabelled antibodies suffer from problems which limit or complicate their use as the therapeutic agents.

Proc. Am. Assoc. Cancer Res. Annu. Meet., volume 29, 1988, page 427 teaches an immunotoxin which is formed by coupling antibody ZME-018 to gelonin using the reagents SPDP and 2-iminothiolane.

US 4894225 discloses that TNF and a suitable immunotoxin when administered simultaneously or in tandem produce a synergistic effect in treating tumour burden in animals.

Summary of the Invention

The present invention provides a composition of matter comprising a conjugate of an antibody (herein designated ZME-018) which recognises the GP 240 antigen on melanoma cancer cells and gelonin, wherein the composition further comprises Tumor Necrosis Factor-α or Interferon-α. One of the antibodies (225.28S) discussed by Wilson et al. (Wilson, et al., Int. J. Cancer 28: 293 (1981)) recognises this melanoma membrane antigen. This antigen is identified therein by the designation GP 240. The antibody 225.28S which binds the GP 240 antigen has been designated and is further referred to herein as ZME-018. The antibody is coupled with gelonin. The cytocidal immunoconjugates are useful to treat and prevent recurrence of tumor-associated GP 240-bearing tumors by administration of these cytocidal immunoconjugates to an individual in need of such treatment.

One of the objects of the present invention is to provide a cytotoxic composition which would specifically bind to and kill tumor cells. Particularly, it is an object of the present invention to provide a cytotoxic composition which would specifically bind to and kill tumor cells which express the GP 240 antigen as described above. Antibody ZME-018 was prepared at Hybritech, Inc. using salt fractionation and DEAE chromatography and was judged homogenous by SDS PAGE (Wilson et al., Int. J. Cancer 28: 293-300 (1981)). Another aspect of the invention concerns the use of the compositions of the invention in the manufacture of a medicament for the treatment of, or the prevention of recurrence of, proliferative cell disease, such as melanoma. Yet another aspect of the invention concerns the use of Interferon-$\alpha$ in the manufacture of a medicament for enhancing the cytotoxic activity of a gelonin conjugated monoclonal antibody.

It is a further object of the present invention to provide such a composition which would be toxic to tumor cells but cause minimal injury to normal tissue.

Description of the Drawings

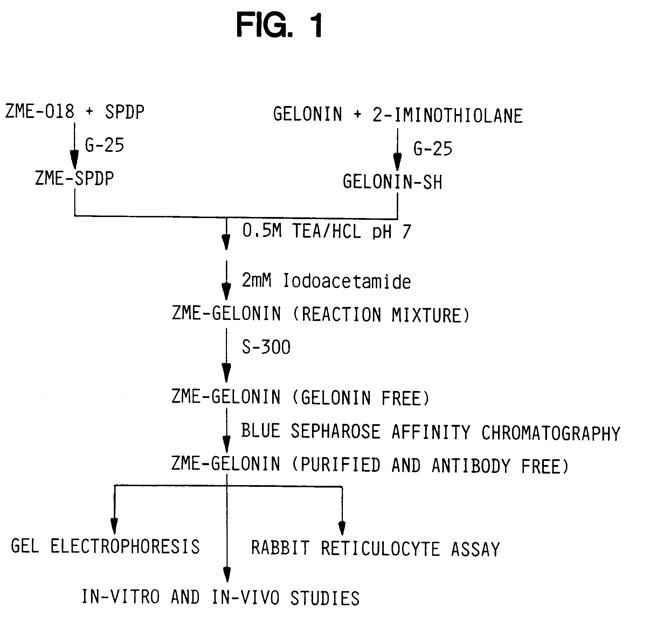

Figure 1 demonstrates the coupling and purification schema for ZME-gelonin.

Figure 2 demonstrates the purification of ZME- gelonin by S-300 gel permeation chromatography.

Figure 3 demonstrates the elution profile of the Cibachron-Blue sepharose column after the high-molecular weight material from S-300 chromatography was applied and eluted with a linear salt gradient (0-300 mM Nac1). Two protein peaks were demonstrated: a flow-through peak (fractions 14-20) and a bound peak eluted with high salt (fractions 44-75).

Figure 4 demonstrates the electrophoretic pattern of gelonin and ZME gelonin conjugate.

Figure 5 demonstrates comparative ELISA Assay data of ZME (open circles) and ZME gelonin (closed circles).

Figure 6 demonstrates the cytotoxicity of ZME- gelonin and free gelonin on log-phase AAB-527 cells after 72 hour exposure.

Figure 7 demonstrates the cytotoxicity of ZME- gelonin and free gelonin on log-phase AAB-527 cells.

Figure 8 demonstrates the cytotoxicity of ZME- gelonin on antigen positive target melanoma cells (AAB-527) and antigen negative T-24 cells in culture.

Figure 9 demonstrates the influence of free antibody on ZME-gelonin cytotoxicity.

Figure 10 demonstrates the effect of IFN-$\alpha$, IFN-$\gamma$ and TNF on ZME-gelonin cytotoxicity. Closed circles show the dose-response for ZME-gelonin alone. Open diamonds show the dose-response for ZME-gelonin plus IFN-$\gamma$. Open triangles show the dose-response for ZME-gelonin in the presence of a fixed amount of TNF-$\alpha$. Closed circles with dotted lines show ZME-gelonin dose-response curve in the presence of a fixed amount of IFN-$\alpha$.

Figure 11 demonstrates the effect of ZME-gelonin on antigen positive (A-375, closed circles) and antigen negative (CEM, open squares) cells in a human tumor stem cell assay.

Figure 12 demonstrates the cytotoxic effect of ZME-gelonin on stem cell survival of different lines obtained from fresh biopsy specimens of 4 different patients.

Figure 13 demonstrates the tissue distribution of ZME antibody and ZME-gelonin conjugate in nude mice bearing human melanoma zenografts.

Detailed Description of the Invention

As used herein the term "monoclonal antibody" means an antibody composition having a homogeneous antibody population. It is not intended to be limited as regards the source of the antibody or the manner in which it is made.

Melanoma cells express a 240 kD (GP 240) antigen on their cell surface. Antibodies to this antigen have been produced. Antibody ZME-018 (from Hybritech, Inc.) is a murine monoclonal antibody IgG$_{2a}$ recognizing a 240 Kd glycoprotein present on most human melanoma cells. Monoclonal antibodies of the IgG$_1$, IgG$_{2a}$ and IgG$_{2b}$ isotypes which recognize an epitope of this 240 kD antigen may be produced. This 240 Kd epitope of the ZME antigen will for the purpose of this invention will be designated the ZME epitope. Thus, all antibodies which recognize this ZME epitope are functionally equivalent.

These representative hybridoma cultures whose cells secrete antibody of the same idiotype, i.e., all recognize the ZME epitope, have been deposited at the American Type Culture Collection of 12301 Parklawn Drive, Rockville, Maryland 20852 ("ATCC").

These monoclonal antibodies may be made by methods known to those of skill in the art. The characterization of and procedure for making the hybridoma cell cultures which produce these antibodies is described in detail. (Wilson et al. Int. J. Cancer 28:293 (1981); Imai et al, Transplant Proc. 12:380-383 (1980)). Briefly, hybridomas were constructed with the murine myeloma cell line Sp2/0-Ag-14 and splenocytes from mice immunized with the melanoma cell line M2l as described by Imai et al., ((1980) Transplant Proc. 12:380-383). The hybridomas secreting the monoclonal antibody (MoAb) 225.28S and 465.12 have been subcloned and are propagated in vitro and in vivo. Both monoclonal antibodies are of the IgG$_{2a}$ subclass and were purified from mouse ascites fluid by absorption/elution from protein A Sepharose 4B (Pharmacia, Piscataway, NJ, USA) prior to their use.

Hybridomas producing antibodies which reacted with human melanoma cells but not with normal human cells were further characterized. The antibodies produced by the ZME cell line and hybridoma producing functionally equivalent antibodies reacted with the ZME antigen on human melanoma cells. They also reacted with 70 - 80% of randomly-obtained melanomas tested, and exhibited no reaction to various tissues as summarized on Table 1 in Example 4.

As used herein with respect to the exemplified murine monoclonal anti-human melanoma antibodies, the term "functional equivalent" means a monoclonal antibody that:

(1) crossblocks an exemplified monoclonal antibody; (b) binds selectively to cells expressing the ZME antigen such as human melanoma cells; (c) has a G or M isotype; (d) binds to the ZME antigen as determined by immunoprecipitation or sandwich immunoassay; and (e) when conjugated to gelonin, exhibits a tissue culture inhibitory dose (TCID) of at least 50% against at least one of the AAB-527, or A375 cell lines when used at a dose of 80-100 units per ml.

Antibody ZME was conjugated to gelonin using N- succinimidyl-3-(2-pyridyldithio)propionate (SPDP) or 2-iminothiolane (IT) as a coupling agent. The conjugates were tested against AAB-527 and A375 cells in a 72-hour tissue culture assay. The antibody conjugates exhibited acceptable antiproliferative activity (TCID 50% of less than 100 units ml) against both of these cell lines.

Further details of the characterization of the antibodies are provided in the examples below.

Immunochemicals

The immunochemical derivatives of this invention that are of prime importance are immunotoxins (conjugates of the ZME antibody and a cytotoxic moiety or a biological response modifier) and labelled (e.g., radiolabelled, enzyme-labelled, or fluorochrome-labelled) derivatives in which the label provides a means for identifying immune complexes that include the labeled antibody.

The cytotoxic moiety of the immunotoxin may be a cytotoxic drug or an enzymatically active toxin of bacterial or plant origin (gelonin), or an enzymatically active fragment ("A chain") of such a toxin. Enzymatically active toxins and fragments thereof are preferred and are exemplified by gelonin, diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytoiacca americana proteins (PAPI, PAPII, and PAP-S), momordica charantia inhibitor, curcin, crotin, saponaria officinalis inibitor, mitogellin, restrictocin, phenomycin, and enomycin. Most preferred is the conjugation with gelonin.

Biological response modifiers which may be coupled to the ZME antibody and used in the present invention include, but are not limited to, lymphokines and cytokines such as IL-1, IL-2, interferons ($\alpha$, $\beta$, or $\gamma$) TNF, LT, TGF-$\beta$, and IL-6. These biological response modifiers have a variety of effects on tumor cells. Among these effects are increased tumor cell killing by direct action as well as increased tumor cell killing by increased host defense mediated processes. Conjugation of antibody ZME to these biological response modifiers will allow selective localization within tumors and, hence, improved anti-proliferative effects while suppressing non-specific effects leading to toxicity of non-target cells.

Cytotoxic drugs which are useful in the present invention include, but are not limited to, adriamycin (and derivatives thereof), cis-platinum complex (and derivatives thereof), bleomycin and methotrexate (and derivatives thereof). These cytotoxic drugs are sometimes useful for clinical management of recurrent tumors and particularly melanoma, but their use is complicated by severe side effects and damage caused to non-target cells. Antibody ZME may serve as a useful carrier of such drugs providing an efficient means of both delivery to the tumor and enhanced entry into the tumor cells themselves. In addition, specific antibody delivery of cytotoxic drugs to tumors will provide protection of sensitive sites such as the liver, kidney and bone marrow from the deleterious action of the chemotherapeutic agents. Use of drugs conjugated to antibody ZME as a delivery system allows lower dosage of the drug itself, since all drug moieties are conjugated to antibodies which concentrate within the tumor.

Conjugates of the monoclonal antibody may be made using a variety of bifunctional protein coupling agents. Examples of such reagents are SPDP, IT, bifunctional derivatives of imidoesters such as dimethyl adipimidate. HCl, active esters such as disuccinimidyl suberate, aldehydes such as glutaraldehyde, bis-azido compounds such as bis(p-azidobenzoyl) hexanediamine, bis-diazonium derivatives such as bis-(p-diazoniumbenzoyl)-ethylenediamine, diisocyanates such as tolylene 2,6-diisocyanate, and bis-active fluorine compounds such as a 1,5-difluoro-2,4-dinitrobenzene.

When used to kill human melanoma cells in vitro for therapeutic or for diagnostic purposes, the conjugates will typically be added to the cell culture medium at a concentration of at least about 10 nM. The formulation and mode of administration for in vitro use are not critical. Aqueous formulations that are compatible with the culture or perfusion medium will normally be used.

Cytotoxic radiopharmaceuticals for diagnosing and treating tumors carrying the ZME antigen such as melanoma may be made by conjugating high linear energy transfer (LET) emitting isotopes to the antibodies. The term "cytotoxic moiety" as used herein is intended to include such isotopes.

The labels that are used in making labeled versions of the antibodies include moieties that may be detected directly, such as fluorochromes and radiolabels as well as moieties, such as enzymes, that must be reacted or derivatized to be detected. Examples of such labels are $^{32}$P, $^{125}$I, $^{3}$H, $^{14}$C, fluorescein and its derivatives, rhodamine and its derivatives, dansyl, umbelliferone, luciferia, 2,3-dihydrophthalzainediones, horseradish peroxidase, alkaline phosphatase, lysozyme, and glucose-6-phosphate dehydrogenase. The antibodies may be tagged with such labels by known methods. For instance, coupling agents such as aldehydes, carbodiimides, dimaleimide, imidates, succinimides, bis-diazotized benzadine and the like may be used to couple the antibodies with the above-described fluorescent, chemiluminescent, and enzyme labels.

The antibodies and labeled antibodies may be used in a variety of immunoimaging or immunoassay procedures to detect the presence of tumors expressing the ZME antigen such as melanoma in a patient or monitor the status of such cancer in a patient already diagnosed to have it. When used to monitor the status of a cancer a quantitative immunoassay procedure may be used. Such monitoring assays are carried out periodically and the results compared to determine whether the patient's tumor burden has increased or decreased. Common assay techniques that may be used include direct and indirect assays. Direct assays involve incubating a tissue sample or cells from the patient with a labeled antibody. If the sample ZME antigen bearing cells includes melanoma cells, the labeled antibody will bind to those cells. After washing the tissue or cells to remove unbound labeled antibody, the tissue sample is read for the presence of labeled immune complexes.

For diagnostic use the antibodies will typically be distributed in kit form. These kits will typically comprise: the antibody in labeled form in suitable containers, reagents for the incubations and washings, and substrates or derivatizing agents depending on the nature of the label. Antigen ZME controls and instructions may also be included.

Administration of the immunotoxins of the present invention to an individual who has been diagnosed as having a tumor with the ZME antigenic determinant will allow targeting and concentration of the cytotoxic agent at the site where it is needed to kill the tumor cells. By so targeting the cytotoxic agents, non-specific toxicity to other organs, tissues and cells will be eliminated or decreased.

When used _in vivo_ for therapy, the immunotoxins are administered to the patient in therapeutically effective amounts (i.e., amounts that eliminate or reduce the patient's tumor burden). They will normally be administered parenterally, preferably intravenously. The dose and dosage regimen will depend upon the nature of the cancer (primary or metastatic) and its population, the characteristics of the particular immunotoxin, e.g., its therapeutic index, the patient, and the patient's history. The amount of immunotoxin administered will typically be in the range of about 0.1 to about 10 mg/kg of patient weight.

For parenteral administration the immunotoxins will be formulated in a unit dosage injectable form (solution, suspension, emulsion) in association with a pharmaceutically acceptable parenteral vehicle. Such vehicles are inherently nontoxic and nontherapeutic. Examples of such vehicles are water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances that enhance isotonicity and chemical stability, e.g., buffers and preservatives. The immunotoxin will typically be formulated in such vehicles at concentrations of about 0.1 mg ml to 10 mg ml.

Gelonin toxin was purified from the seeds of <u>gelonium multiflorum</u> by the method of Stirpe, et al. Briefly, gelonin was extracted from the seeds by homogenization in buffered saline solution (pH 7.4). The supernatant was concentrated after dialysis against 5 mM sodium phosphate (pH 6.5) and the gelonin further purified by ion exchange chromatography as described in Example 1. The purity of the gelonin toxin was assessed by high pressure liquid chromatography (HPLC) and sodium dodecylsulphate-polyacylamide gel electrophoreseis (SDS-Page). Gelonin toxin migrated as a single band with an approximate molecular weight of 29-30,000 daltons.

Gelonin toxin activity was measured as described in Example 2 by protein synthesis inhibition in a cell-free system.

Antibody ZME-018 modified with SPDP as described in Example 5 was conjugated with iminothiolane modified gelonin as described in Examples 3 and 6. The gelonin conjugated antibody was purified as described in Example 7 by column chromatography on a Sephadex G-75 column.

The toxicity of the gelonin-conjuated antibody was determined by protein synthesis inhibition and its antiproliferative activity was determined by <u>in vitro</u> and <u>in vivo</u> tests.

The following examples provide a detailed description of the preparation, characterization, and use of the immunotoxin monoclonal antibodies of this invention. These examples are not intended to limit the invention in any manner.

## Example 1

### Purification of Gelonin

Seeds of Gelonium multiflorum were shelled and the nuts ground in a homogenizer with eight volumes of 0.14 M NaCl containing 5 mM sodium phosphate (pH 7.4). The homogenate was left overnight at 4°C. with continuous stirring, cooled on ice and centrifuged at 35,000 times g for 20 minutes at 0°C. The supernatant was removed, dialyzed against 5 mM sodium phosphate (pH 6.5) and concentrated using a pm10 filter. The sample was layered on a CM-52 ion-exchange column (20 x 1.5 cm) equilibrated with 5 mM sodium phosphate (pH 6.5). Material which bound to the ion exchange resin was eluted with 400 ml of 0 to 0.3 M linear NaCl gradient at a rate of 25 ml hour at 4°C. Five ml fractions were collected. The fractions were monitored at 280 nm in a spectrophotometer. The gelonin eluted in about fractions 55-70 and was the last major elution peak. Fractions 55-70 were pooled, dialyzed against double distilled water and concentrated by lyophilization. The purity and the molecular weight of each preparation was checked on high pressure liquid chromotography using a TSK 3000 gel permeation column with 50 mM sodium phosphate buffer, pH 7.4 and 15% sodium dodecylsulphate-polyacrylamide gel electrophoresis (SDS-page). Gelonin migrated as a single band with an approximate molecular weight of 29-30,000 daltons.

## Example 2

### Assay of Gelonin Activity

The gelonin activity was monitored in a cell-free protein synthesis inhibition assay. The cell-free protein synthesis inhibition assay was performed by sequentially adding to 50 $\mu$l rabbit reticulocyte lysate, thawed immediately before use, mixing after each addition, the following components: 0.5 ml of 0.2 M Tris HCl (pH 7.8), 8.9 ml of ethylene glycol, and 0.25 ml of 1 M HCl).

Twenty microliters of a salt-amino acid-energy mixture (SAEM) consisting of: 0.375 M KCl, 10 mM Mg(CH$_3$CO$_2$)$_2$, 15 mM glucose, 0.25-10 mM amino acids (excluding leucine), 5 mM ATP, 1 mM GTP, 50 mM Tris-HCl (pH 7.6), 10 ul Creatinine phosphate-creatinine phosphokinase, 8 ul $^{14}$C leucine (Amersham, 348 mCi mmol), and adding 1.5 ul of solutions containing varying concentrations of the gelonin mixture. The mixture was incubated for 60 minutes at 30°C. $^{14}$C-leucine incorporation was monitored in an aliquot of the mixture by precipitating synthesized protein on glass fiber filters, washing in 10% TCA and acetone, and monitoring the radioactivity in a Beta-counter using Aquasol scintillation fluid. Gelonin with a specific activity no lower than 4 x 10$^9$ U/mg was used for conjugation with the antibodies. A unit of gelonin activity is the amount of gelonin protein which causes 50% inhibition of incorporation of [$^{14}$C] leucine into protein in the cell free assay.

## Example 3

### Modification of Gelonin With Iminothiolane

Gelonin in phosphate buffered saline was concentrated to approximately 2 milligrams/ml in a Centricon 10 microconcentrator. Triethanolamine hydrochloride (TEA/HCl), pH 8.0 and EDTA were added to a final concentration of 60mM TEA/HCl and 1mM EDTA pH 8.0. 2-Iminothiolane stock solution (20mM) was added to a final concentration of 1 mM and the sample was incubated for 90 minutes at 4°C. under a stream of nitrogen gas.

Excess iminothiolane was removed by gel filtration on a column of Sephadex G-25 (1 x 24cm) pre-equilibrated with 5 mM bis-tris acetate buffer, pH 5.8 containing 50 mM NaCl and 1 mM EDTA. Fractions were analyzed for protein content in microtiter plates using the Bradford dye binding assay. Briefly, forty microliters of sample, 100 ul of phosphate buffered saline (PBS) and 40 ul of dye concentrate were added to each well. Absorbance at 600mm was read on a Dynatech Microelisa Autoreader. Gelonin elutes at the void volume (about fractions 14-20). These fractions are pooled and concentrated by use of a Centricon-10 microconcentrator.

## Example 4

### Preparation and Characterization of Monoclonal Antibody to ZME Melanoma Antigen

### Antibody-Secreting Hybridomas

One 8-week-old female BALB/c St mouse (SCRF Breeding Colony, La Jolla, Calif.) was injected intraperitoneally (i.p.) with 10$^7$ human melanoma M21 cells and boosted i.p. with 5 x 10$^6$ M21 cells 2 weeks later. One 50-week-old male NZB/B mouse (SCRF Breeding Colony) was primed with 5 x $10^6$ BW5 melanoma cells and boosted with 5 injections of 5 x $10^6$ BW5, M51, Colo 38, BW5 and M21 melanoma cells at monthly intervals. Three days after the booster injection, the mouse was sacrificed, and the spleen was removed, splenocytes were dissociated with a scalpel to make a cell suspension. The spleen cell suspension was treated with 0.17 $\partial$ $NH_4Cl$ in 0.01 M Tris, pH 7.2, for 10 min to lyse the red blood cells. Then, these splenocytes were fused with SP2/0Ag14 cells as described by Gefter et al. ((1977) Somat. Cell Genet. 3:231.) with the following minor modifications: 5 x $10^7$ spleen cells and $10^7$ SP2/0Ag14 cells were hybridized with 0.3 ml of 30% (v/v) polyethylene glycol 1000 (PEG) (Baker Chemical Co., Phillipsburg, N.J.) in MEM. After incubation with PEG the cells were washed, and cultured at a concentration of 2x $10^6$ cells/ml in D-MEM overnight. The next day, the cells were suspended in 40 ml HAT medium and pipetted into about 400 wells (0.1 ml/well) of microtiter plates (Costar #3596, Cambridge, Mass.) One drop (approximately 25ml) of HAT medium was added at weekly intervals. After 2-3 weeks, the hybridomas selected for further studies were cultured in D-MEM with 10% FBS. Hybridomas were expanded in tissue culture and were grown in the peritoneal cavity of BALB/c mice primed with 0.5 ml of Pristane (Pfaltz and Bauer, Inc., Stamford, Conn.). The spent culture medium and ascitic fluid were used as source of antibody.

Clones of the hybridoma were grown in vitro according to known tissue culture techniques such as is described by Cotten, et al., Eur. J. Immunol. 3: 136 (1973).

Hybridomas producing antibodies which reacted with human melanoma cells but not with normal human cells were further characterized.

As shown on Table 1, they did not react with most normal tissues tested. The antibodies produced by the ZME cell line and hybridomas-producing functionally equivalent antibodies reacted with the ZME antigen on human melanoma cells such as M-21.

TABLE 1

| NORMAL TISSUE REACTIVITY OF ANTIBODY ZME-018 (225.285)* | |
| --- | --- |
| TISSUE | REACTIVITY** |
| Bladder | 0/3 |
| Brain Cortex | 0/2 |
| Cartilage | 0/2 |
| Colon | 0/2 |
| Nipples | 1/2 |
| Duodenum | 0/1 |
| Endometrium | 0/1 |
| Kidney | 0/2 |
| Liver | 0/1 |
| Lung | 0/4 |
| Lymph Node | 0/3 |
| Mammary Gland | 0/3 |
| Ovary | 0/1 |
| Pancreas | 0/1 |
| Peripheral Blood Lymphocytics | 0/4 |
| Peripheral Nerve | 0/1 |
| Prostate | 0/2 |
| Salivary Gland | 0/3 |
| Skeletal Muscle | 0/1 |
| Skin | 0/5 |
| Spleen | 0/1 |
| Stomach | 0/3 |
| Thyroid | 0/2 |
| Tonsil | 0/1 |
| Testes | 0/5 |

*From P. Giacomini, et al. <u>Cancer Research</u> 44:1281-1287, 1984

**Number of samples antigen positive/ number samples tested.

<u>Example 5</u>

<u>Modification of Monoclonal Antibody ZME-018 With SPDP</u>

N-succinimidyl 3-(2-pyridyldithio) (propionate)(SPDP) in dimethylformamide was prepared as a stock solution of 3 mg ml in dry dimethylforamide. Since the crystalline SPDP can undergo hydrolysis, the actual concentration of chemically reactive crosslinker was determined by spectrophotometric methods by analyzing the absorbance at 260 nm in a dual-beam spectrophotometer. The concentration of SPDP stock is calculated from the following equation:

$$\frac{\text{Change in absorbance (260nm)}}{0.02 \times 103 \text{ ml mmol}} \times \frac{(3.01)}{0.01} = \text{mmoles/ml/SPDP}$$

One milligram of monoclonal antibody ZME in 1.0 ml of phosphate buffered saline (PBS) was added to a glass tube. SPDP stock solution was slowly added at about a 5-fold molar excess to the tube (approximately 10 ul of stock solution), mixing constantly. The mixture was incubated for 30 minutes at room temperature, mixing every 5 minutes during the incubation period.

Excess unreacted SPDP was removed from the sample by gel filtration chromatography on a Sephdex G-25 column (1 x 24 cm) pre-equilibrated with 100 mM sodium phosphate buffer pH 7.0 containing 0.5 mM EDTA (Buffer A). Fractions (0.5 ml) were collected and analyzed for protein content using the Bradford dye binding assay (Bradford, Anal. Biochem. 72: 248-254 (1976)). Absorbance (600 nm) was monitored in a 96-well plate using a Bio-TEK Microplate autoreader. Antibody eluted at the void volume (fractions 14-20) and these fractions were pooled and kept at 4°C. The protein was concentrated in a Centricon-30 microcentrator. The Centricon retentate was washed with 100 mM sodium phosphate buffer, pH 7.0 containing EDTA (0.5 mM). The antibody was concentrated to a final volume of approximately 0.5-0.75 ml.

## Example 6

### Conjugation of SPDP-Modified Monoclonal Antibody ZME-018 With Iminothiolane-modified Gelonin

One milligram of purified gelonin (2 mg/ml in PBS) prepared as described in Example 1 was modified with iminothiolane as described in Example 3. Monoclonal antibody ZME modified as described in Example 4 was mixed with an equal weight of gelonin modified as in Example 3. This proportion corresponded to a 5-fold molar excess of gelonin as compared to antibody. The pH of the mixture was adjusted to 7.0 by the addition of 0.05 M TEA/HCl buffer pH 8.0 and the mixture was incubated for 20 hours at 4°C under nitrogen. Iodoacetamide (0.1 M) was added to a final concentration of 2 mM to block any remaining free sulfhydryl groups and incubation was continued for an additional hour at about 25°C. The reaction mixture was stored at 4°C. until purification by gel filtration.

## Example 7

### Purification of Gelonin-Monoclonal Antibody 15A8 Complexes

Non-conjugated gelonin and low molecular weight products were removed from the reaction mixtures of Example 6 by gel filtration on a Sephadex S-300 column (1.6 x 31 cm) pre-equilibrated with PBS.

Reaction mixtures from Example 6 were concentrated to approximately 1 ml with a Centricon 30 microconcentrator before loading on the Sephadex column. The column was washed with PBS. One ml fractions were collected and 50 ul aliquots are analyzed for protein by the Bradford dye binding assay (Bradford, Anal. Biochem 72: 248 (1976)).

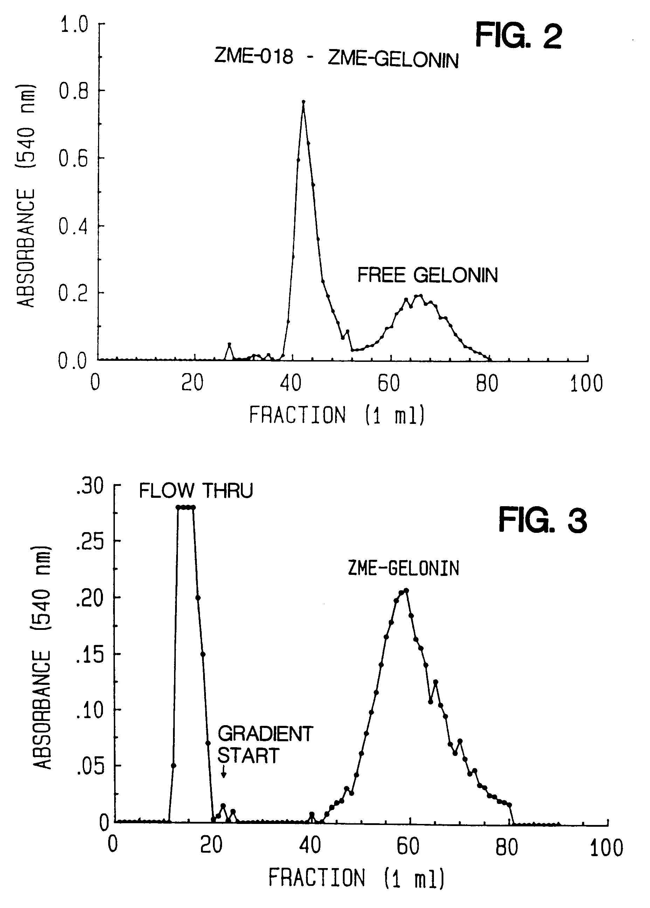

As shown on Figure 2, free- and gelonin-conjugated antibody eluted in the void volume (about fractions 28-40) while, unconjugated gelonin elutes at about fractions 45-65.

To remove unconjugated ZME-018, the high molecular peak (fraction 28-40) from the S-300 column was applied to an affinity chromatography column of Blue Sepharose CL-6B (1 x 24 cm) pre-equilibrated with 10 mM phosphate buffer (pH 7.2) containing 0.1 M NaC1. After sample loading, the column was washed with 30 ml of buffer to completely elute non-conjugated antibody. The column was eluted with a liner salt gradient of 0.1 to 2 M NaC1 in 10 mM phosphate buffer pH 7.2. Protein content of the eluted fractions was determined by the Bradford dye-binding assay.

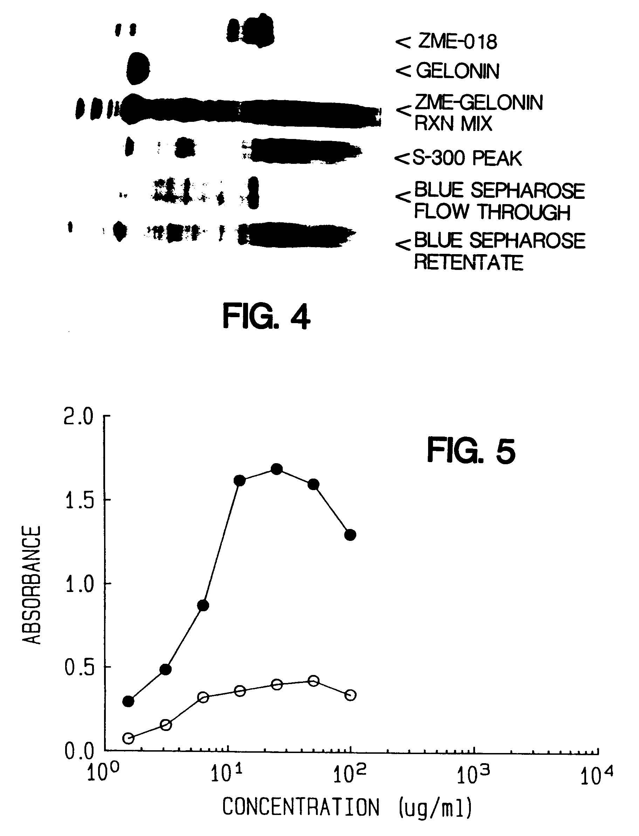

Figure 2 demonstrates the elution profile of the S- 300 column and demonstrates that gelonin can be separated from gelonin-antibody conjugate and unconjugated antibody, both of which coelute in the first peak (about fractions 28-40). This elution pattern was confirmed by electrophoresis of 50 ul aliquots on 5-20% gradient non-reducing SDS polyacrylamide gels as shown on Figure 4. The coupling mixture was loaded on lane 3. Bands for free gelonin (lane 2), free antibody (lane 1) and for one molecule of gelonin coupled per molecule of antibody and two molecules of gelonin coupled per antibody molecule are shown. The void volume peak of the S-300 column containing free antibody and antibody-gelonin conjugate was loaded on lane 4.

Non-conjugated antibody was removed from the gelonin conjugated antibody by affinity chromatography on a column (1 x 24 cm) of Blue Sepharose CL-6B pre-equilibrated with 10 mM phosphate buffer, pH 7.2 containing 0.1 M NaCl. After loading the S-300 eluate sample, the column was washed with 30 ml of the same buffer to completely elute non-conjugated antibody.

Gelonin-conjugated antibody bound to the column and was eluted with a linear salt gradient of 0.1 to 2 M NaCl in 10 mM phosphate buffer, pH 7.2. The antibody- gelonin complex eluted at approximately 0.7 M NaCl as shown on Figure 3 which depicts the elution profile of the Blue Sepharose column. Protein content of the eluted fractions was determined by the Bradford dye binding assay. The protein-containing fractions were pooled and the elution pattern confirmed by electrophoresis on a 5 to 20% gradient non-reducing polyacrylamide gel. The electrophoretic pattern of the ZME-gelonin complex is shown on Figure 4. The flow-through peak (fractions 14-20) contains only free antibody (Fig. 4, lane 5) while fractions 50-80, eluted with high salt, contain ZME-gelonin conjugate free of unconjugated gelonin or antibody (Fig. 4, lane 6). The final product contained ZME antibody coupled to 1, 2 and 3 gelonin molecules. Average gelonin content was 1.5 molecules per antibody molecule.

The rabbit reticulocyte in vitro translation system described in Example 2 was utilized to estimate the gelonin activity of the essentially pure gelonin-ZME antibody complex. One unit of activity in this assay was defined as the amount of protein required to provide 50% inhibition of protein synthesis as compared to untreated controls. Utilizing this assay, the specific activity of both the native gelonin and the ZME-gelonin conjugate were determined to be $2 \times 10^8$ U/mg and $8.2 \times 10^5$ U/mg respectively. The essentially pure gelonin-ZME antibody is active in the reticulocyte lysate assay. A 1:1000 dilution of the original sample caused approximately a 50% inhibition of protein synthesis, i.e., a 50% reduction of the incorporation of $^{14}$C-leucine into protein. Thus, the activity of the original preparation was 1000 U/ml.

## EXAMPLE 8

### Cell Culture Methods

ZME antigen-negative human bladder carcinoma (T-24) human cervical carcinoma or ZME antigen-positive human metastatic melanoma tumor cells A375M or AAB-527 were maintained in culture using minimal essential medium supplemented (MEM) with 10% heat-inactivated fetal bovine serum plus 100 mM non-essential amino-acids, 2mM L-glutamine, 1mM sodium pyruvate, vitamins and antibiotics. Cultured cells were routinely screened and found free of mycoplasma infection.

### A. Cell Proliferation Assay

Cell lines were maintained in culture in complete medium at 37°C in a 5% $CO_2$-humidified air incubator. For assays with combinations of TNF, immunotoxins, IFN$\alpha$, and IFN$\gamma$, cultures were washed, detached using versene, and resuspended in complete medium at a density of $25 \times 10^3$ cells/ml. Two hundred ml aliquots were dispensed into 96-well microtiter plates and the cells were then allowed to adhere. This results in a sparsely seeded population of cells. After 24 hours the media were replaced with media containing different concentration of either immunotoxins, toxins, TNF, IFNq, or IFNa. The cells were incubated for 72 hours and analyzed for relative cell proliferation by crystal violet staining.

### B. Crystal Violet Staining

Cells were washed 3 times with PBS containing calcium and magnesium fixed and stained with 20% (v/v) methanol containing 0.5% (v/v) crystal violet. Bound dye was eluted with 150 ul of Sorenson's citrate buffer (0.1M sodium citrate, pH 4.2-50% (v/v ethanol) for 1 hour at room temperature. The absorbance was measured at 600 nm using a Bio-Teck microplate reader. Relative cell proliferation (RCP) was calculated as follows:

$$RCP = \frac{\text{Mean Absorbance (Drug Treated)}}{\text{Mean Absorbance (Non-drug Treated)}} \times 100$$

### C. Human Tumor Colony Assay

Tumor biopsy specimens were obtained from melanoma patients during clinically indicated biopsy procedures. Tumor cell suspensions were prepared aseptically (Leibovitz, et al., Int. J. Cell Cloning 1: 478-485 (1983)). Additionally, the A375P melanoma and the CEM leukemia cell lines from the American Type Culture collection (Rockville, MD) were also tested. Testing for the effects of ZME-gelonin on the fresh melanoma cell suspensions and cell lines was assessed in HTCA using standardized procedures for tumor cell plating in semisolid medium (agarose) in the presence of complete medium containing 10% fetal calf serum, each 0.5 ml culture plate containing 100,000 cells for fresh tumors and 10,000 cells for the cell lines (Hamburger, et al., Science 197: 461-463 (1977); Salmon, et al., N. Engl. J. Med. 298: 1321-1327 (1978); Salmon, et al., J. Clin. Oncol. 7: 1346-1350 (1989)). ZME-gelonin prepared as described above was tested by addition to the culture plates shortly after tumor cell plating. ZME-gelonin was added to triplicate plates at each of four concentrations 0.025 ng ml to 250 ng ml. In addition to untreated control plates, unconjugated ZME-18 monoclonal antibody and free gelonin were tested in parallel. Cell lines and tumor cell cultures were incubated for an average of 10 days at 37°C in 5% $CO_2$ in air in a humidified incubator, and colony formation evaluation with a viability stain (Shoemaker, et al., Cancer Res. 45: 2145-2153 (1985)) and an automated image analysis instrument optimized for colony counting (Salmon, et al., Int. J. Cell Cloning 2: 142-160 (1984)). Percent survival of ZME-018 treated cultures in relation to simultaneous untreated controls were determined in the same experiments. Dose-response curves were then plotted graphically.

## Example 9

### Comparison of Binding of Gelonin-conjugated and Unconjugated ZME Antibody to Target Cells

The ability of the gelonin-conjugated and unconjugated ZME antibody to bind to target cells was assessed. The binding of ZME-gelonin immunotoxin to antigen positive (AAB-527 cells) or antigen negative (T-24 cells) was tested by ELISA assay.

Fifty thousand target cells (AAB-527 cells) or non-target (T-24 cells) were added to each well of microtiter plate. The cells were dried on the plates overnight at 37°C. The cells were then washed with three changes of cold PBS and air dried overnight. The cell surface antigenic determinants remain antigenically active after this treatment.

After attachment of the cells, the plates were washed with Washing Buffer (9.68 g Tris, 64.8 g sodium chloride, 16 ml Tween 20, 800 mg thimerasol in 8 l of double distilled water). Antibody samples were diluted in Washing Buffer containing 1% bovine serum albumin (w/v) (Diluting buffer). Fifty microliters of various concentrations ranging from .02 to 50 ug/ml of either conjugated or unconjugated ZME antibody were added to the wells. After incubation for 1 hour at 4°C, the supernatants are removed and the wells washed twice with Washing Buffer.

Fifty microliters per well of horseradish peroxidase conjugated goat anti-mouse IgG obtained from Bio-Rad and diluted 1:1000 (v/v) (HPGAM) in Diluting buffer was added to each well. The plates were incubated for 1 hour at 4°C and the wells washed twice with Washing Buffer. After incubation of the plates with 50 ul of Substrate Solution (80 mM citrate phosphate (pH 5.0), 1 mM 2,2'AZINO-Bis (3-ETHYL BENZ-THIAZOLINE-6-SULFONIC ACID) (ABTS) DIAMMONIUM SALT (SIGMA CHEMICAL CO) and 4 ul of 30% hydrogen peroxide) in the dark for 30 minutes at room temperature, 25 ul of 4 N sulfuric acid was added to each well. The absorbance at 492 nm was determined on an Elisa reader.

As shown in Figure 5, both native ZME and the ZME gelonin conjugate bound well to target cells after 60 minute exposure. Surprisingly, the ZME-gelonin conjugate bound target cells better than did the native antibody. This increase was not due to modification of the antibody by SPDP since SPDP-modified ZME behaved identically to that of native ZME. The increase was also not due to binding of target cells to the gelonin portion of the molecule since pre-treatment of target cells with native gelonin had no effect on either antibody or immunotoxin binding.

Neither ZME nor ZME-gelonin bound to antigen negative T-24 cells as estimated by ELISA assay.

## Example 10

### Cytotoxicity of Gelonin and Gelonin-ZME Antibody Complex

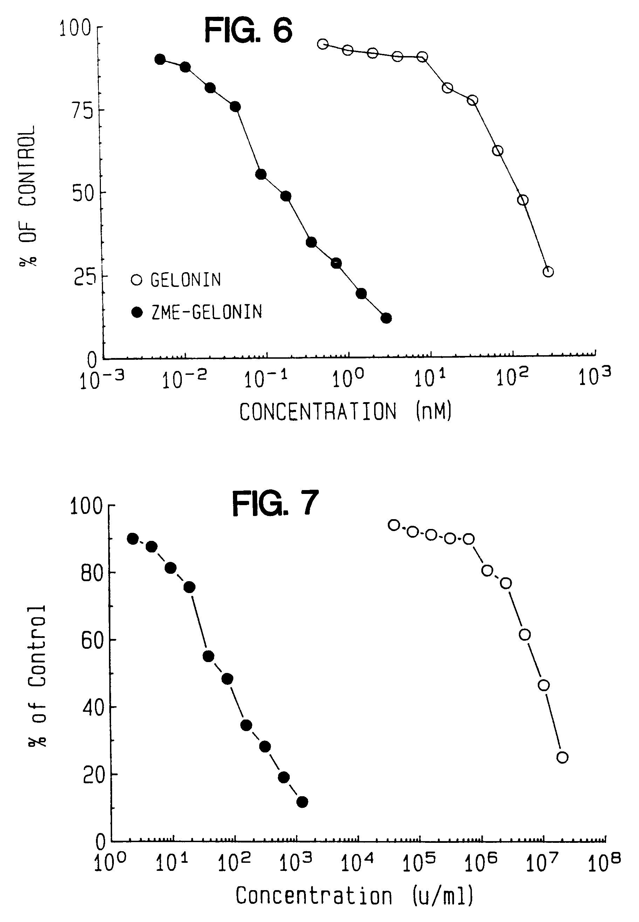

Cytotoxicity studies of the ZME-gelonin conjugate were performed on antigen-positive cells after continuous (72 hour) exposure to the immunotoxin or native gelonin. As shown in Figure 6, when antigen-positive AAB-527 cells were exposed to approximately 0.1/nM ZME gelonin, 50% cell death was observed. When cells were exposed to native gelonin, a concentration of 100 nM gelonin was required to reduce the cell number to 50% of the untreated control.

Target cells were then treated with various concentrations of ZME-gelonin or gelonin alone on a unit basis as determined in Example 2. As shown in Figure 7, 50% cytotoxicty was obtained using 50 units/ml of ZME-gelonin conjugate while $1 \times 10^7$ units/ml of the free gelonin were required to achieve the same effect.

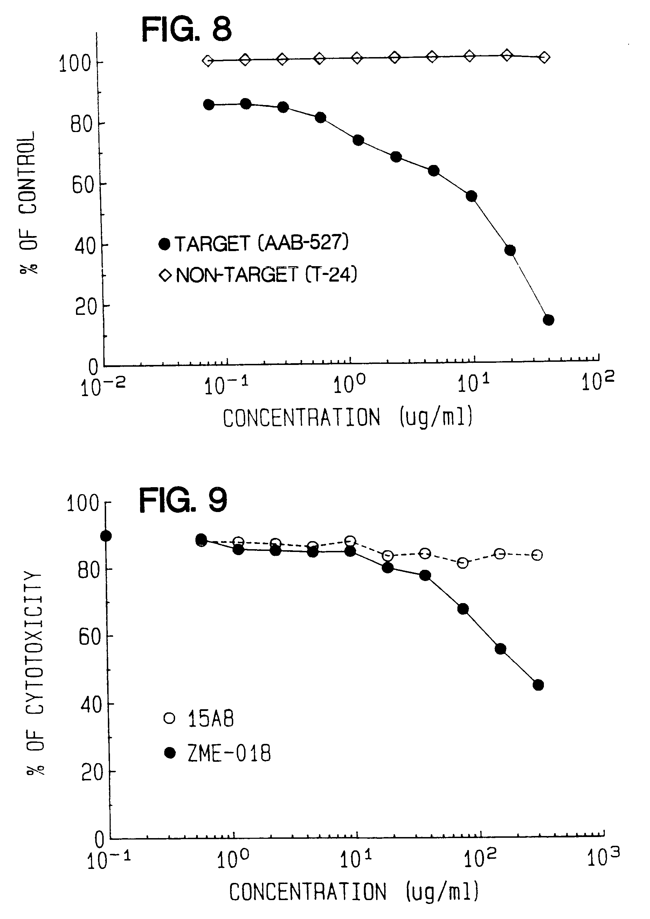

The effect of ZME-gelonin was determined against antigen-negative T-24 cells in log-phase culture. As shown in Figure 8, gelonin alone produced 50% cytotoxicity in AAB-S27 cells at a concentration of 100 ug/ml, similar to that found on AAB-S27 cells. ZME-gelonin produced 50% cytotoxicity in target T-24 cells at a concentration of 10 $\mu$g/ml. However, the ZME-gelonin immunotoxin was not cytotoxic against non-target T-24 cells even at the highest concentration tested.

In order to further demonstrate that the ZME gelonin cytotoxicity was mediated through the ZME cell surface antigen, a fixed dose of ZME-gelonin which achieves 80% cytotoxicity was added to target log phase melanoma cells in culture in the presence of free ZME antibody or an irrelevant antibody (15A8, an antibody that does not bind to melanoma cells). As shown on Figure 9, the presence of increasing amounts of ZME antibody suppressed the cytotoxicity of the ZME-gelonin conjugate while the 15A8 antibody had no effect. Thus, the cytotoxicity of the ZME-gelonin conjugate was directly mediated by the binding of the ZME antibody to ZME antigen on the target cell.

## EXAMPLE 11

### Modulation of ZME-gelonin cytotoxicity with IFN $\alpha$, IFN $\gamma$ and TNF.

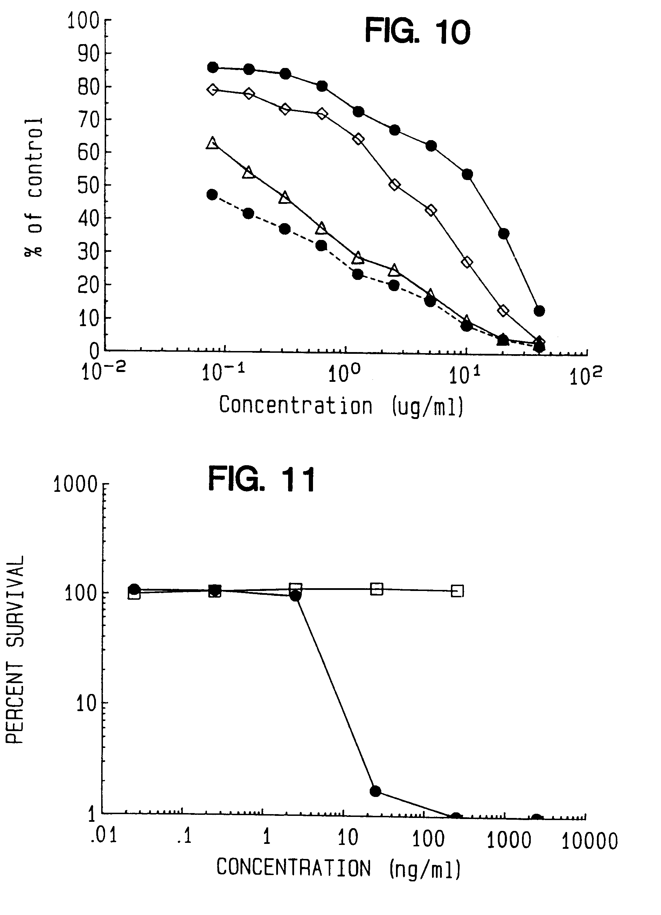

To demonstrate the effects of treatment with various biological response modifiers on immunotoxin cytotoxicity, log-phase melanoma cells were treated for 24 hours with fixed doses of IFN $\alpha$ (200 u/ml), IFN$\gamma$ (20,000 u/ml) or rTNF-$\alpha$ (20,000 u/ml). These doses were previously determined to have minimal effect (approximately 20%) cytotoxic effect against these cells. The cells were then treated for 72 hours with various doses of ZME-gelonin. As shown in Figure 10, treatment with rIFN$\gamma$ resulted in a 2-fold increase in sensitivity to the ZME-gelonin immunotoxin. However, pre-treatment with both rIFN$\alpha$ and TNF both resulted in a 2-log increase in sensitivity to the immunotoxin. Addition of fixed doses of rIFN$\alpha$, rIFN$\gamma$ or rTNF to antigen-positive cells resulted in augmented cytotoxicity of the ZME-gelonin toxin. Treatment with rTNF-$\alpha$ caused the greatest increase in immunotoxin cytotoxicity followed by rIFN$\alpha$ and rIFN$\gamma$.

Substantial augmentation of ZME-gelonin cytotoxic effect was observed with pre-treatment of rIFN$\alpha$ and rTNF but not with rIFN$\gamma$. While it has been observed that IFN$\alpha$ and IFN-$\gamma$ can up-regulate some melanoma surface antigens such as P-97, there was little effect of the agent on the high molecular weight antigen (GP 240) recognized by ZME (Murray, et al., Proc. Am. Assoc. Cancer Res. 27: 313 (1986); Greiner, et al., Cancer Res. 44: 3208-3214 (1984); Greiner, et al. Cancer Res. 46: 4984-4990 (1986); Groacomini, et al., J. Immunol. 133: 1649-1655 (1984); Imai, et al., J. Immunol. 127: 505-509 (1981); Murray, et al., J. Biol. Res. Mod. 7: 152-161 (1988).). Therefore, the mechanism of TNF$\alpha$ and IFN$\alpha$ induced augmentation of ZME-gelonin activity is not clear but could involve changes in the antibody internalization rate, changes in the cellular processing of the immunotoxin or a modulation of any one of several interferon-mediated enzymes.

Since only cells containing the ZME antigen on their surface were killed by the gelonin ZME immunotoxin, this immunotoxin is an efficient method to target and kill ZME tumor associated antigen containing cells while minimizing or preventing damage or injury to normal non-tumor associated antigen-bearing cells.

Example 12

Effect of ZME-Gelonin Immunotoxin in Human Tumor Colony Assay (HTCA)

The activity of ZME-gelonin was also assessed using the human tumor colony assay against cells obtained from biopsy of four patients with melanoma.

In vitro cytotoxicity against human cells in culture was also assessed in the HTCA described in Example 8C. Various doses of ZME-gelonin immunotoxin were added to an antigen positive (A-375 melanoma) and antigen negative CEM cell lines. Survival of colonies was assessed 72 hours after addition. As shown in Figure 11, doses of immunotoxin between 0.25 and 2500 ng ml resulted in almost complete supression of colony survival of the antigen-positive cell line (closed circle). ZME-018 and free gelonin alone or combined together were not cytotoxic. There was no effect against the antigen-negative line (CEM) even at the highest concentration of immunotoxin tested (open squares).

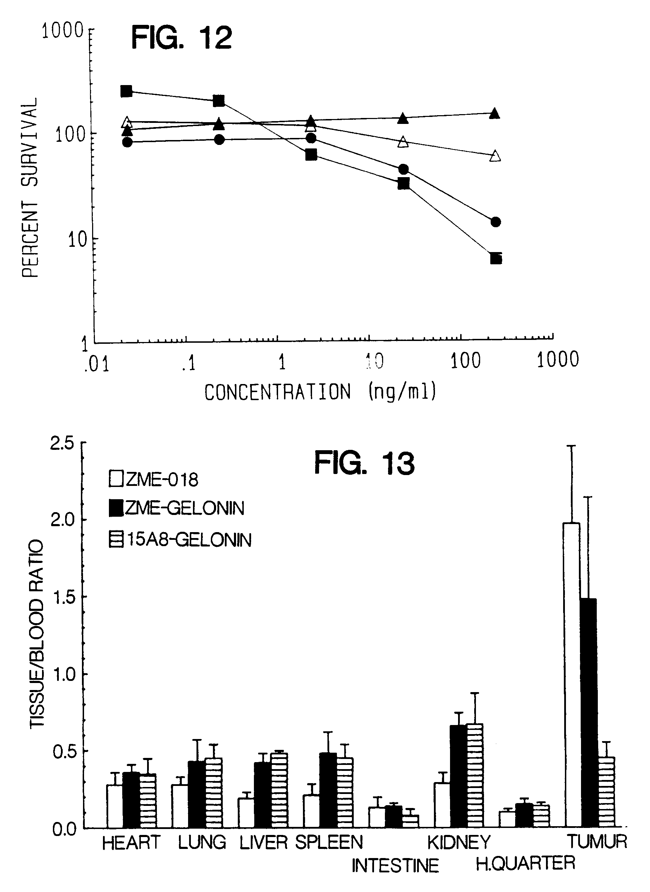

The effect of ZME-gelonin against 4 different fresh biopsy specimens is shown in Figure 12. Eighty to 90% reduction in survival of melanoma colony forming cells was found in two specimens at the highest immunotoxin dose tested (250 ng ml). One patient showed 50% inhibition of cell growth at this dose, while one patient showed no cytotoxicity of the immuno-conjugate. Only a modest (25%) reduction in colony number was noted with a third specimen. Growth enhancement was noted in the fourth sample at the highest immunotoxin dose. In addition, growth enhancement was observed in one specimen at low doses, while higher doses produced substantial cytotoxicity. As in the cell line experiments, addition of unconjugated ZME-018 and free gelonin were not cytotoxic at the doses tested.

While the HTSCA assay is not infallable, approximately 75% of clinically active antitumor agents are positive in this test system. Agents inactive in the HTSCA have thus far, proven inactive clinically. Therefore, acivity of the ZME-gelonin conjugate in the HTSCA has a 75% probability of demonstrating positive clinical value.

Example 13

Tissue Distribution of ZME Antibody

The tissue distribution of $^{125}$I labeled ZME antibody was compared to relevant immunotoxin (ZME-gelonin) and an irrelevant immunotoxin (15A8-gelonin). Each antibody or antibody-conjugate was administered intravenously in the tail vein to 5 nude mice bearing human melanoma xenografts. Each animal received 10mg of total protein labeled with 0.5 $\mu$Ci of $^{125}$I in a total volume of 100$\mu$l of phosphate-buffered saline.

As shown in Figure 13, the irrelevant 15A8-gelonin conjugate did not localize specifically in tumor tissues (T/B ration 0.5). In contrast, both the relevant ZME and ZME-gelonin conjugate demonstrated specific localization (T/B ratios of 2.0 and 1.5 respectively). There was no statistically significant difference in the uptake of $^{125}$I into tumor after ZME or ZME-gelonin administration. One skilled in the art will readily appreciate that the present invention is well adapted to carry out the objects and obtain the ends and advantages mentioned, as well as those inherent therein. The compounds, methods, procedures and techniques described herein are presently representative of the preferred embodiments, are intended to be exemplary, and are not intended as limitations on the scope of the present invention.

Changes therein and other uses will occur to those skilled in the art which are encompassed within the spirit of the invention and are defined by the scope of the appended claims.

**Claims**

**Claims for the following Contracting States : AT, BE, CH, DE, DK, FR, GB, IT, LI, LU, NL, SE**

1. A composition of matter comprising a conjugate of ZME-018 antibody and gelonin, wherein said composition further comprises Tumor Necrosis Factor-$\alpha$.

2. A composition of matter comprising a conjugate of ZME-018 antibody and gelonin, wherein said composition further comprises Interferon-$\alpha$.

3. Use of a composition of claim 1 or claim 2 in the manufacture of a medicament for the treatment of, or the prevention of recurrence of, proliferative cell disease.

4. Use as claimed in claim 3, wherein the proliferative cell disease is melanoma.

5. Use of Interferon-$\alpha$ in the manufacture of a medicament for enhancing the cytotoxic activity of a gelonin conjugated monoclonal antibody.

6. Use as claimed in claim 5 wherein said antibody is selected from the group consisting of an antibody directed against a cell surface antigen of melanoma cells, a cell surface antigen of breast carcinoma cells, and a cell surface antigen of cervical cancer cells.

7. Use as claimed in claim 6 wherein said antibody is ZME-018.

**Claims for the following Contracting State : ES**

1. A process for preparing a composition of matter comprising a conjugate of ZME-018 antibody and gelonin, wherein said composition further comprises Tumor Necrosis Factor-$\alpha$, which process comprises forming the composition from its components.

2. A process for preparing a composition of matter comprising a conjugate of ZME-018 antibody and gelonin, wherein said composition further comprises Interferon-$\alpha$, which process comprises forming the composition from its components.

3. Use of a composition of claim 1 or claim 2 in the manufacture of a medicament for the treatment of, or the prevention of recurrence of, proliferative cell disease.

4. Use as claimed in claim 3, wherein the proliferative cell disease is melanoma.

5. Use of Interferon-$\alpha$ in the manufacture of a medicament for enhancing the cytotoxic activity of a gelonin conjugated monoclonal antibody.

6. Use as claimed in claim 5 wherein said antibody is selected from the group consisting of an antibody directed against a cell surface antigen of melanoma cells, a cell surface antigen of breast carcinoma cells, and a cell surface antigen of cervical cancer cells.

7. Use as claimed in claim 6 wherein said antibody is ZME-018.

**Claims for the following Contracting State : GR**

1. A process for preparing a composition of matter comprising a conjugate of ZME-018 antibody and gelonin, wherein said composition further comprises Tumor Necrosis Factor-$\alpha$, which process comprises forming the composition from its components.

2. A process for preparing a composition of matter comprising a conjugate of ZME-018 antibody and gelonin, wherein said composition further comprises Interferon-α, which process comprises forming the composition from its components.

3. A composition of matter comprising a conjugate of ZME-018 antibody and gelonin, wherein said composition further comprises Tumor Necrosis Factor-α.

4. A composition of matter comprising a conjugate of ZME-018 antibody and gelonin, wherein said composition further comprises Interferon-α.

5. Use of a composition of claim 3 or claim 4 in the manufacture of a medicament for the treatment of, or the prevention of recurrence of, proliferative cell disease.

6. Use as claimed in claim 5, wherein the proliferative cell disease is melanoma.

7. Use of Interferon-α in the manufacture of a medicament for enhancing the cytotoxic activity of a gelonin conjugated monoclonal antibody.

8. Use as claimed in claim 7 wherein said antibody is selected from the group consisting of an antibody directed against a cell surface antigen of melanoma cells, a cell surface antigen of breast carcinoma cells, and a cell surface antigen of cervical cancer cells.

9. Use as claimed in claim 8 wherein said antibody is ZME-018.

**Patentansprüche**

**Patentansprüche für folgende Vertragsstaaten : AT, BE, CH, DE, DK, FR, GB, IT, LI, LU, NL, SE**

1. Zusammensetzung umfassend ein Konjugat aus einem ZME-018-Antikörper und Gelonin, wobei die Zusammensetzung weiterhin Tumor-Nekrose-Faktor α enthält.

2. Zusammensetzung umfassend ein Konjugat aus einem ZME-018-Antikörper und Gelonin, wobei die Zusammensetzung weiterhin Interferon α enthält.

3. Verwendung einer Zusammensetzung nach Anspruch 1 oder Anspruch 2 zur Herstellung eines Arzneimittels zur Behandlung oder Verhütung des Wiederauftretens einer proliferativen Zellerkrankung.

4. Verwendung nach Anspruch 3, worin die proliferative Zellerkrankung ein Melanom ist.

5. Verwendung von Interferon α zur Herstellung eines Arzneimittels zur Verbesserung der cytotoxischen Aktivität eines mit Gelonin konjugierten monoklonalen Antikörpers.

6. Verwendung nach Anspruch 5, wobei der Antikörper ausgewählt ist aus der Gruppe bestehend aus einem Antikörper, der gegen ein Zelloberflächenantigen von Melanomzellen, ein Zelloberflächenantigen von Brustkarzinomzellen und ein Zelloberflächenantigen von Gebärmutterhalskrebszellen gerichtet ist.

7. Verwendung nach Anspruch 6, worin der Antikörper ZME-018 ist.

**Patentansprüche für folgenden Vertragsstaat : ES**

1. Verfahren zur Herstellung einer Zusammensetzung umfassend ein Konjugat aus ZME-018-Antikörper und Gelonin, wobei die Zusammensetzung weiterhin Tumor-Nekrose-Faktor α enthält, wobei das Verfahren umfaßt, daß man die Zusammensetzung aus ihren Komponenten bildet.

2. Verfahren zur Herstellung einer Zusammensetzung umfassend ein Konjugat aus ZME-018-Antikörper und Gelonin, wobei die Zusammensetzung weiterhin Interferon α enthält, wobei das Verfahren umfaßt, daß man die Zusammensetzung aus ihren Komponenten bildet.

3. Verwendung einer Zusammensetzung nach Anspruch 1 oder Anspruch 2 zur Herstellung eines Arzneimittels zur Behandlung oder Verhütung des Wiederauftretens einer proliferativen Zellerkrankung.

4. Verwendung nach Anspruch 3, worin die proliferative Zellerkrankung ein Melanom ist.

5. Verwendung von Interferon α zur Herstellung eines Arzneimittels zur Verbesserung der cytotoxischen Aktivität eines mit Gelonin konjugierten monoklonalen Antikörpers.

6. Verwendung nach Anspruch 5, worin der Antikörper ausgewählt ist aus der Gruppe bestehend aus einem Antikörper, der gegen ein Zelloberflächenantigen von Melanomzellen, ein Zelloberflächenantigen von Brustkarzinomzellen und ein Zelloberflächenantigen von Gebärmutterhalskrebszellen gerichtet ist.

7. Verwendung nach Anspruch 6, worin der Antikörper ZME-018 ist.

**Patentansprüche für folgenden Vertragsstaat : GR**

1. Verfahren zur Herstellung einer Zusammensetzung umfassend ein Konjugat aus ZME-018-Antikörper und Gelonin, wobei die Zusammensetzung weiterhin Tumor-Nekrose-Faktor α enthält, wobei das Verfahren umfaßt, daß man die Zusammensetzung aus ihren Komponenten bildet.

2. Verfahren zur Herstellung einer Zusammensetzung umfassend ein Konjugat aus ZME-018-Antikörper und Gelonin, wobei die Zusammensetzung weiterhin Interferon α enthält, wobei das Verfahren umfaßt, daß man die Zusammensetzung aus ihren Komponenten bildet.

3. Zusammensetzung umfassend ein Konjugat aus ZME-018-Antikörper und Gelonin, wobei die Zusammensetzung weiterhin Tumor-Nekrose-Faktor α enthält.

4. Zusammensetzung umfassend ein Konjugat aus ZME-018-Antikörper und Gelonin, wobei die Zusammensetzung weiterhin Interferon α enthält.

5. Verwendung einer Zusammensetzung nach Anspruch 3 oder Anspruch 4 zur Herstellung eines Arzneimittels zur Behandlung oder Verhütung des Wiederauftretens einer proliferativen Zellerkrankung.

6. Verwendung nach Anspruch 5, worin die proliferative Zellerkrankung ein Melanom ist.

7. Verwendung von Interferon α zur Herstellung eines Arzneimittels zur Verbesserung der cytotoxischen Aktivität eines mit Gelonin konjugierten monoklonalen Antikörpers.

8. Verwendung nach Anspruch 7, worin der Antikörper ausgewählt ist aus der Gruppe bestehend aus einem Antikörper, der gegen ein Zelloberflächenantigen von Melanomzellen, ein Zelloberflächenantigen von Brustkarzinomzellen und ein Zelloberflächenantigen von Gebärmutterhalskrebszellen gerichtet ist.

9. Verwendung nach Anspruch 8, worin der Antikörper ZME-018 ist.

**Revendications**

**Revendications pour les Etats contractants suivants : AT, BE, CH, DE, DK, FR, GB, IT, LI, LU, NL, SE**

1. Composition comprenant un conjugué d'anticorps ZME-018 et de gélonine, ladite composition comprenant en outre le facteur de nécrose tumorale α.

2. Composition comprenant un conjugué d'anticorps ZME-018 et de gélonine, ladite composition comprenant en outre l'interféron α.

3. Utilisation d'une composition de la revendication 1 ou de la revendication 2 dans la préparation d'un médicament pour le traitement ou la prévention de la récurrence d'une maladie cellulaire proliférative.

4. Utilisation selon la revendication 3 dans laquelle la maladie cellulaire proliférative est un mélanome.

**5.** Utilisation de l'interféron $\alpha$ dans la préparation d'un médicament pour accroître l'activité cytotoxique d'un anticorps monoclonal conjugué à la gélonine.

**6.** Utilisation selon la revendication 5 dans laquelle ledit anticorps est choisi dans le groupe constitué par un anticorps dirigé contre un antigène de surface cellulaire des cellules de mélanome, un antigène de surface cellulaire des cellules de carcinome du sein et un antigène de surface cellulaire des cellules de cancer cervical.

**7.** Utilisation selon la revendication 6 dans laquelle ledit anticorps est ZME-018.

**Revendications pour l'Etat contractant suivant : ES**

**1.** Procédé de préparation d'une composition comprenant un conjugué d'anticorps ZME-018 et de gélonine, ladite composition comprenant en outre le facteur de nécrose tumorale $\alpha$, ce procédé comprenant la formation de la composition à partir de ses composants.

**2.** Procédé de préparation d'une composition comprenant un conjugué d'anticorps ZME-018 et de gélonine, ladite composition comprenant en outre l'interféron $\alpha$, ce procédé comprenant la formation de la composition à partir de ses composants.

**3.** Utilisation d'une composition de la revendication 1 ou de la revendication 2 dans la préparation d'un médicament pour le traitement ou la prévention de la récurrence d'une maladie cellulaire proliférative.

**4.** Utilisation selon la revendication 3 dans laquelle la maladie cellulaire proliférative est un mélanome.

**5.** Utilisation de l'interféron $\alpha$ dans la préparation d'un médicament pour accroître l'activité cytotoxique d'un anticorps monoclonal conjugué à la gélonine.

**6.** Utilisation selon la revendication 5 dans laquelle ledit anticorps est choisi dans le groupe constitué par un anticorps dirigé contre un antigène de surface cellulaire des cellules de mélanome, un antigène de surface cellulaire des cellules de carcinome du sein et un antigène de surface cellulaire des cellules du cancer cervical.

**7.** Utilisation selon la revendication 6 dans laquelle ledit anticorps est ZME-018.

**Revendications pour l'Etat contractant suivant : GR**

**1.** Procédé de préparation d'une composition comprenant un conjugué d'anticorps ZME-018 et de gélonine, ladite composition comprenant en outre le facteur de nécrose tumorale $\alpha$, ce procédé comprenant la formation de la composition à partir de ses composants.

**2.** Procédé de préparation d'une composition comprenant un conjugué d'anticorps ZME-018 et de gélonine, ladite composition comprenant en outre l'interféron $\alpha$, ce procédé comprenant la formation de la composition à partir de ses composants.

**3.** Composition comprenant un conjugué d'anticorps ZME-018 et de gélonine, ladite composition comprenant en outre le facteur de nécrose tumorale $\alpha$.

**4.** Composition comprenant un conjugué d'anticorps ZME-018 et de gélonine, ladite composition comprenant en outre l'interféron $\alpha$.

**5.** Utilisation d'une composition de la revendication 3 ou de la revendication 4 dans la préparation d'un médicament pour le traitement ou la prévention de la récurrence d'une maladie cellulaire proliférative.

**6.** Utilisation selon la revendication 5, dans laquelle la maladie cellulaire proliférative est le mélanome.

**7.** Utilisation de l'interféron $\alpha$ dans la préparation d'un médicament pour accroître l'activité cytotoxique d'un anticorps monoclonal conjugué à la gélonine.

**8.** Utilisation selon la revendication 7 dans laquelle ledit anticorps est choisi dans le groupe constitué par un anticorps dirigé contre un antigène de surface cellulaire de cellules de mélanome, un antigène de surface cellulaire de cellules de carcinome du sein, et un antigène de surface cellulaire de cellules de cancer cervical.

9. Utilisation selon la revendication 8 dans laquelle ledit anticorps est ZME-018.

# FIG. 1

```
ZME-018 + SPDP                    GELONIN + 2-IMINOTHIOLANE
      │ G-25                               │ G-25
      ▼                                    ▼
   ZME-SPDP                           GELONIN-SH
         └─────────────────┬──────────────┘
                           │ 0.5M TEA/HCL pH 7
                           ▼
                           │ 2mM Iodoacetamide
                           ▼
              ZME-GELONIN (REACTION MIXTURE)
                           │ S-300
                           ▼
              ZME-GELONIN (GELONIN FREE)
                           │ BLUE SEPHAROSE AFFINITY CHROMATOGRAPHY
                           ▼
           ZME-GELONIN (PURIFIED AND ANTIBODY FREE)
         ┌─────────────────┼──────────────┐
         ▼                 │              ▼
GEL ELECTROPHORESIS        │     RABBIT RETICULOCYTE ASSAY
                           ▼
             IN-VITRO AND IN-VIVO STUDIES
```

FIG. 2

FIG. 3

< ZME-018

< GELONIN

< ZME-GELONIN RXN MIX

< S-300 PEAK

< BLUE SEPHAROSE FLOW THROUGH

< BLUE SEPHAROSE RETENTATE

## FIG. 4

## FIG. 5

FIG. 6

○ GELONIN
● ZME-GELONIN

FIG. 7

FIG. 8

● TARGET (AAB-527)
◇ NON-TARGET (T-24)

FIG. 9

○ 15A8
● ZME-018

FIG. 10

FIG. 11

FIG. 12

FIG. 13